# EUROPEAN PATENT SPECIFICATION

(11) **EP 2 776 115 B1**
(45) Date of publication and mention of the grant of the patent: **13.03.2019**
(21) Application number: 12848717.0
(22) Date of filing: 07.11.2012
(51) Int. Cl.: A61M 35/00, A61K 9/00, A61M 5/28

(54) **MEDICINAL PATCH AND INJECTOR SYSTEM**
MEDIZINISCHES PFLASTER UND INJEKTORSYSTEM
TIMBRE MÉDICINAL ET SYSTÈME D'INJECTEUR

(30) Priority: 07.11.2011 US 201161556542 P
(43) Date of publication of application: 17.09.2014
(73) Proprietor: Eli Lilly and Company, Indianapolis, IN 46285 (US)
(72) Inventor: ULRICH, Michael, Scott, Columbus, OH 43204 (US); SWEENEY, Andrew, Vankirk, Columbus, OH 43215 (US)
(74) Representative: J A Kemp
(86) International application number: PCT/US2012/063874
(87) International publication number: WO 2013/070705

(56) References cited:
- WO-A1-97/28750
- WO-A1-2007/071485
- WO-A1-2009/116045
- WO-A1-2009/144726
- US-A1- 2007 228 071
- US-A1- 2009 131 860
- US-A1- 2009 259 176
- US-A1- 2010 174 225
- US-A1- 2012 078 216

## Description

### BACKGROUND

The present disclosure relates to medical devices. It finds particular application in conjunction with an injector system, and will be described with particular reference thereto.

Hypodermic syringes are often used to deliver selected doses of medication to patients. Such hypodermic syringes generally include a barrel and a plunger mounted for reciprocating movement within the barrel. A needle is mounted to the barrel and includes a cannula for receiving/delivering medication. Medication to be injected with the hypodermic syringe often is stored in a vial having a pierceable elastomeric seal. Medication in the vial is accessed by piercing the elastomeric seal with the needle. A selected dose of the medication is drawn into the barrel by withdrawing the plunger to create a vacuum in the barrel that is then filled by the medication. The needle is then withdrawn from the vial, inserted into a patient, and the medication is injected by moving the plunger in the opposite direction to expel the medicine from the barrel.

Some medications, such as insulin, are usually self-administered. Medication delivery pens have been developed to facilitate the self-administration of medication. Such delivery pens attempt to simplify both the delivery and dosing of the medication to make self-administering as reliable as administration by a healthcare professional. In addition, such devices often include various safety features to prevent or diminish the possibility of needle contamination and/or the needle making unintended contact with the user or another person.

To this end, various devices have been developed that simplify self-administration of medication. Reusable devices may accommodate a replaceable medication cartridge and include an injection mechanism for injecting a prescribed dose of medicine. Some devices are capable of varying the dosage size. Disposable pen devices generally have a fixed dosage of medication, and are designed for a single use. Many pen devices include safety mechanisms, such as a retracted needle that is shielded from inadvertent contact until deployed for injection.

WO 2007/071485 A1 discloses a skin retention device for a medical jet-injection unit comprising a contact face provided with adhesive to be applied in contact with the skin of a subject prior to an injection. The contact face is further provided with at least one injection opening. The adhesive substantially surrounds the injection opening(s) in the proximity of the opening(s), such that skin retention against the contact face close to the injection opening(s) is ensured.

WO 2009/144726 A1 discloses a system and a device for sustained infusion of fluids. A skin securable dispensing patch comprises a reusable part, a disposable part and a cradle unit.

US 2007/228071 A1 discloses a device for dispensing fluid, the device comprising: a patch-sized housing; a pump enclosed in the housing; and a dispensing assembly, downstream of the pump and in fluid communication with it.

### BRIEF DESCRIPTION

According to the present invention there is provided the medication administration system of claim 1.

In accordance with one aspect of the present disclosure, an injection system includes a wearable component (e.g., a patch device) adapted to be worn by user, and a medication delivery component (e.g., an injection device) configured to cooperate with the wearable component to deliver medication to the user. The wearable component can provide a target area to the user for targeting the medication delivery component during use, and further serve to prepare the injection site to minimize the potential for injection site reactions. Both the wearable component and the medication delivery component can include electronics and/or other devices for detecting and/or communication information regarding usage of the injection system including dose amount, frequency, time, etc. The system can be configured to communicate such information to a third party, such as a health care professional.

In accordance with another aspect, a medication administration system comprises a wearable component adapted to be worn on a surface of a user's skin, and an injection component configured to cooperate with the wearable component to administer a medication to the user. The wearable component can include an adhesive patch for adhering the wearable component to the user's skin. The wearable component can be adapted to apply pain management to an injection site before an injection. The wearable component can include a housing having an opening for receiving the injection component.

The wearable component can further include a monitoring device for monitoring at least one parameter related to the use of at least one of the wearable component and the injection component. The monitoring device can include at least one sensor for sensing activity associated with use of at least one of the wearable component and the injection component, and/or a communication interface for communicating with the injection component. The communication interface can include an RFID reader, and the injection device can include an RFID transponder configured to be read by the RFID reader of the wearable component. The monitoring device can also include a communication interface for communicating data to a third party device, such as a mobile phone, a computer, an internet connected device or other remote device. A cover for covering at least a portion of the opening in the housing when the injection component is not received therein can be provided. The wearable component can include a user interface having at least one of a user input device, a display, or a sound producing device.

The injection component can include a housing, a portion of said housing adapted to be received in the opening in the housing of the wearable device. The injection device can also include an injection mechanism operative to inject medication into a user's body. At least one sensor associated with the injection device can be provided for detecting a parameter related to injection of medication via the injection mechanism.

In accordance with another aspect, a wearable medical device comprises a housing having a surface for engaging an outer surface of a user's skin, an opening in the housing for receiving an associated medication delivery device for injecting medication into a user's body, and a monitoring device for monitoring at least one parameter related to the use of at least one of the wearable component and the injection component.

These and other non-limiting characteristics are more particularly described below.

### BRIEF DESCRIPTION OF THE DRAWINGS

The following is a brief description of the drawings, which are presented for the purpose of illustrating the exemplary embodiments disclosed herein and not for the purpose of limiting the same.
**Figure 1** is a perspective view of an exemplary injection system in accordance with the disclosure.
**Figure 2** is a schematic block diagram of the exemplary injection system of **Figure 1****.**
**Figure 3** is a schematic block diagram of another exemplary injection system in accordance with the disclosure.
**Figure 4** is an illustration showing an injection device and a patch device in another exemplary embodiment of the present disclosure.
**Figure 5** is a perspective view of a patch device of the present disclosure prior to being applied to the user.
**Figure 6** is a top view of the patch device, illustrating the removal of the tab to expose the adhesive for applying the patch device to the user.
**Figure 7** is a side view of an injection device of the present disclosure.
**Figure 8** is a side view showing the injection device interacting with the patch device during medication administration.

### DETAILED DESCRIPTION

A more complete understanding of the processes and apparatuses disclosed herein can be obtained by reference to the accompanying drawings. These figures are merely schematic representations based on convenience and the ease of demonstrating the existing art and/or the present development, and are, therefore, not intended to indicate relative size and dimensions of the assemblies or components thereof.

Although specific terms are used in the following description for the sake of clarity, these terms are intended to refer only to the particular structure of the embodiments selected for illustration in the drawings, and are not intended to define or limit the scope of the disclosure. In the drawings and the following description below, it is to be understood that like numeric designations refer to components of like function. In the following specification and the claims which follow, reference will be made to a number of terms which shall be defined to have the following meanings.

The singular forms "a," "an," and "the" include plural referents unless the context clearly dictates otherwise.

Numerical values in the specification and claims of this application should be understood to include numerical values which are the same when reduced to the same number of significant figures and numerical values which differ from the stated value by less than the experimental error of conventional measurement technique of the type described in the present application to determine the value.

All ranges disclosed herein are inclusive of the recited endpoint and independently combinable (for example, the range of "from 2 grams to 10 grams" is inclusive of the endpoints, 2 grams and 10 grams, and all the intermediate values). The endpoints of the ranges and any values disclosed herein are not limited to the precise range or value; they are sufficiently imprecise to include values approximating these ranges and/or values.

As used herein, approximating language may be applied to modify any quantitative representation that may vary without resulting in a change in the basic function to which it is related. Accordingly, a value modified by a term or terms, such as "about" and "substantially," may not be limited to the precise value specified, in some cases. In at least some instances, the approximating language may correspond to the precision of an instrument for measuring the value. The modifier "about" should also be considered as disclosing the range defined by the absolute values of the two endpoints. For example, the expression "from about 2 to about 4" also discloses the range "from 2 to 4."

The systems and methods of the present disclosure can be used with both manual syringes or auto-injectors and is not limited to cylindrical geometries. For the purposes of this disclosure, the term "injection device" is used to refer to both manual syringes and auto-injectors of any size or shape.

Certain disease treatment routines require frequent, sometimes daily, injections. Persons administering self-injection routines are often advised to rotate the injection site using four to six different sites. Frequent injections and side effects from medications can cause adverse reactions at injection sites. The present disclosure provides a system approach for self injection. The medication administration system is comprised of two main components; a device to administer injections, and a device to prepare the injection site to minimize the potential for injection site reactions.

As will be described in more detail below, the components of the system components contain electronics sensors, components, and/or wireless communication devices for the purpose of providing dose information and other usage data to software applications for reviewing dose history and making treatment management decisions.

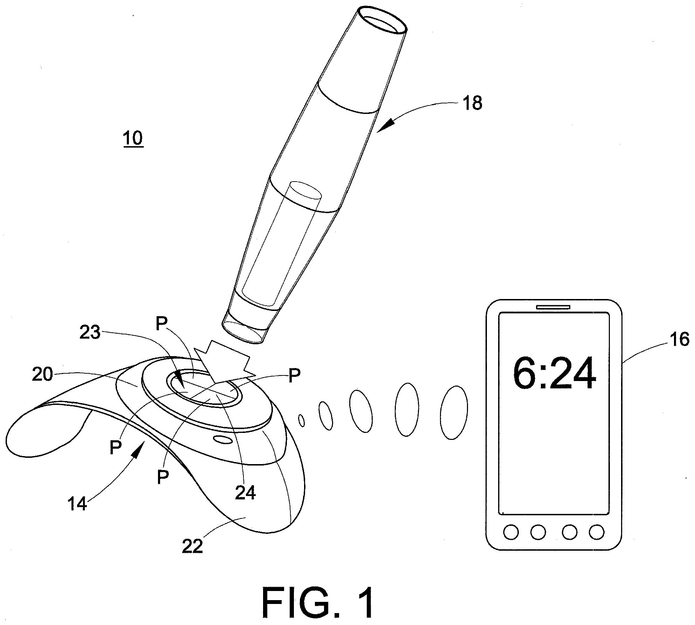

With reference to **Figure 1****,** an exemplary medication delivery system in accordance with the disclosure is illustrated and identified generally by reference numeral **10.** The system **10** generally comprises a wearable component in the form a patch device **14,** and an injection component in the form of an injection device **18** for dispensing medication. The patch device **14** is configured to transmit data associated with usage of the system to a third party device, such as smart phone **16,** for example.

The patch device **14** is applied to the skin, using an adhesive, for example, at a predetermined injection site. The patch device **14** can contain medication to reduce pain from administering injections and/or to prepare the injection site to minimize side effects caused by frequent injections. The patch device **14** is a semi-durable device worn by the user for a short period of time, possibly up to two weeks, or more. The patch device **14** contains a target receptacle that provides an interface area for the user to inject medication through using an injection device, such as injection device **18.** When the useful period of the patch device **14** expires, the device is removed from the skin and disposed. A new patch device may then be applied to the user for continuing the electronic management of medication.

The patch device **14** contains electronics and wireless communication devices and sensor mechanisms to provide additional treatment management features and capabilities to the end user. Sensors are incorporated into the patch device **14** for the purpose of communicating with the injection device **18,** and for communicating with a software application that resides on a personal electronic device, such as a smartphone, iPad, computer or the like. The treatment information monitored and transmitted by the patch device can include the specific medicine contained in the injection device, the dose amount, time/date stamp of the injection, patient ID, etc.

The patch device **14** includes a housing **20,** which is depicted here as being disc shaped. However, it should be noted that the housing may be of any desired shape. A flexible patch **22** is connected to the housing **20** and enables the wearable component to be secured to a user's body, such as an arm, a leg, a portion of the torso or any other suitable location for injecting medication. The patch **22** can be coated with a suitable adhesive for adhering the patch device **14** to a surface of the user's skin. As will be appreciated, the flexibility of the patch **22** allows the patch device **14** to be worn on a wide variety of body parts and also facilitates user movement of body parts on which the patch device **14** is secured. For example, a patch device **14** secured to a user's forearm or bicep may be subjected to stress due to expansion and contraction of the user's muscles during everyday activities. The flexible patch **22** accommodates such expansion and contraction while maintaining adhesion to the surface of the user's skin.

The housing **20** of the wearable component (patch device **14**) as noted above can be disc-shaped and includes an opening **23** that is configured to receive the injection device **18** during a medication injection procedure. The opening is usually centrally located. The opening **23** can be closed by a cover **24** that, as illustrated here, is designed to part upon insertion of the injection device **18** into the opening 23. As such, the injection site can be shielded from exposure to the environment by the housing 20 and cover 24 before and after an injection procedure.

The cover 24 is comprised of four portions P (e.g., flaps) that meet together at a central location within the opening 23. Each of these portions P are flexible and secured about the circumference of the opening 23 such that, upon insertion of the injection device 18 into the opening 23, the portions P deflect downwardly to create an aperture at the center of the opening 23 through which the injection device **18** can deliver the medication. Upon withdrawal of the injection device **18** from the opening **23** in the patch device **14,** the portions P resiliently return to their original position thereby closing off the opening **23** and shielding the injection site from the environment.

As noted, the wearable component **14** can also be configured to deliver and/or apply pain management to the injection site prior to an injection procedure. For example, the patch **22** of the patch device **14** can be saturated with (or otherwise be designed to deliver) a topical anesthetic for numbing the injection site. As will be appreciated, it may be advantageous to apply the topical anesthetic to only a portion of the patch **22,** such as the portion adjacent the opening **23.** The patch device can also be configured to deliver or apply anti-inflammatory or other drug types for managing the injection site so as to prolong the viability of a given injection site.

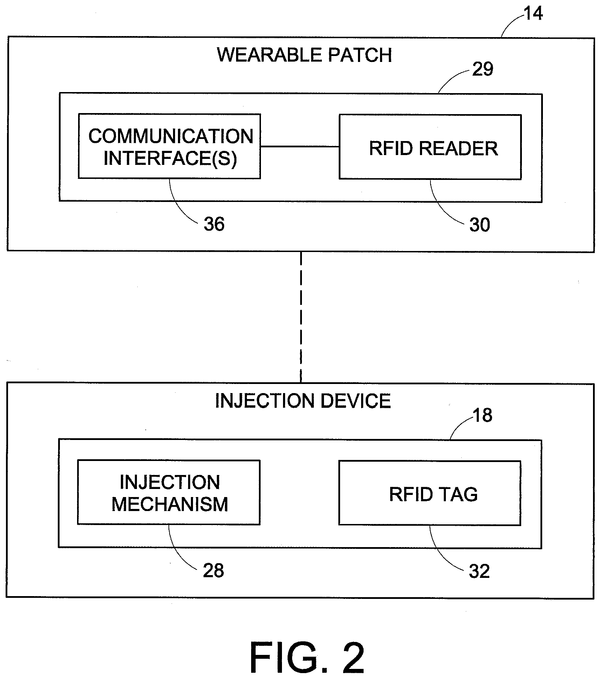

Turning now to the details of the injection device **18,** it will be appreciated that the exemplary device is generally a pen-shaped structure. The injection device **18** has an operative end that is configured to be received in the opening **23** of the patch device **14** (as indicated by the arrow) to deliver medication to the user. To this end, the injection device **18** includes an injection mechanism **28** (see **Figure 2**) that is operative to inject medication into a user's body, such medication being stored in an medicine reservoir or the like.

In some embodiments, the injection device can be a single-use injection device containing a reservoir for liquid medication, a needle and an injection mechanism, and user interface for activation. Onboard sensors and electronic components may be embedded in the injection device for wireless communication with the patch device. Some of the types of information that may be transmitted include device ID number and dose information including amount and time stamp, for example. As another example, for injection devices that can vary the dosage size, the patch device can be used to confirm that the correct dosage is being applied to reduce overdosing.

Although the illustrated injection device **18** is designed to work in conjunction with the patch device **14,** it will be appreciated that other injection components not necessarily designed to operate with the wearable component of the present disclosure can be used with the patch device **14.** However, in such cases, some of the features that will be described below may not be available to a user using an existing rejection device. The details of the injection component insofar as they relate to the injection of medication into a user's body through the user's skin are not necessarily germane to the present disclosure and therefore the details of such mechanisms have been omitted for the sake of brevity. It will be appreciated, however, that a wide variety of injection mechanisms can be used without departing from the scope of the present disclosure.

As noted above, the patch device **14** further includes a monitoring device **29** (or devices) for monitoring at least one parameter related to the use of the patch device **14** and/or the injection device **18.** The monitoring device may include one or more electrical components and or mechanical switches designed to sense or detect certain events or activity associated with use of the system **10.**

For example, referring now to **Figure 2****,** in one basic configuration the patch device **14** can include an RFID reader **30** for interrogating an RFID tag **32** associated with the injection device **18.** A communication interface **36,** which may be a wireless communication interface (WiFi, Bluetooth, etc.) is provided for establishing a communication link between the patch device **14** and another device or devices, such as a smart phone as shown in **Figure 1****,** and/or cell phones, laptops and other like devices. The communication interface **36** can be used for communicating data relating to the usage of the system **10** to another device or system

The RFID reader **30** can be configured to interrogate the RFID tag **32** during an injection procedure. For example, a switch or other mechanical or electrical device can be provided on the patch device **14** for detecting when the injection device **18** is inserted into the opening **23.** When such an event is detected, the patch device **14** can be configured to interrogate the RFID tag **32** and to receive information therefrom such as medicine type, dosage amount etc. This information, along with other information such as time/date etc. can be communicated to a third party device via the communication interface **40.**

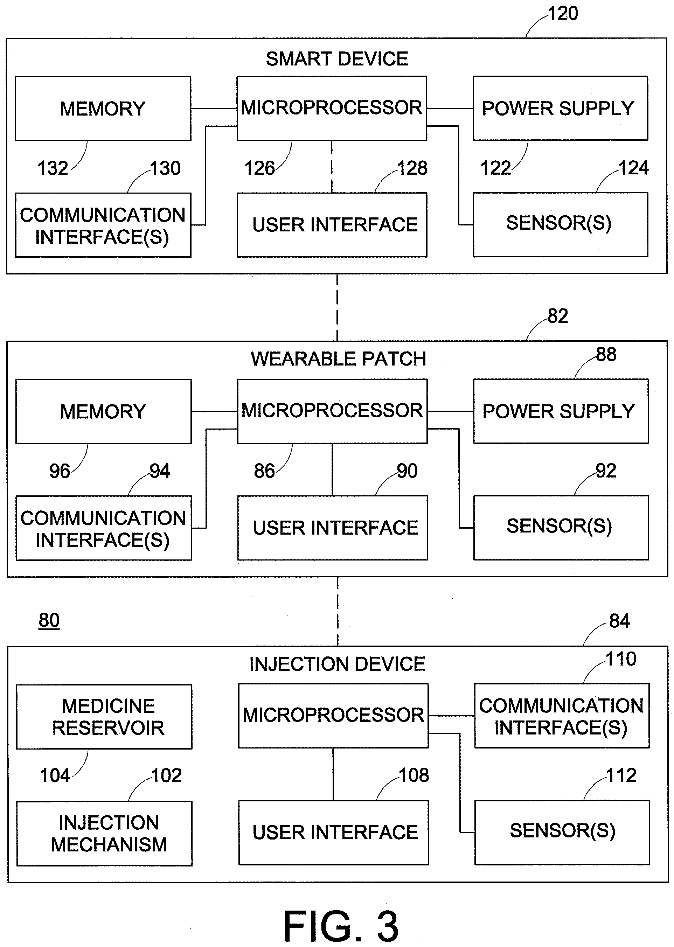

**Figure 3** illustrates a somewhat more sophisticated system **80** including a wearable component **82** and an injection component **84.** Here, the wearable component includes a plurality of electrical components designed to work together to sense one or more parameters related to the use of the system and/or report or otherwise communicate data relating to the sensed parameters to a third party smart device **120** such as a personal computer, a smartphone, a tablet, a laptop, or any other similar device.

As illustrated here, the wearable component **82** includes a microprocessor **86** connected to a power supply **88,** which may be an onboard battery, AC power supply or the like. The wearable component **82** also includes a user interface **90,** one or more sensors **92** for detecting activity relating to usage of the system **80,** and a communication interface **94** for communicating with the injection device **84** and/or a third party or remote device such as those listed above. Onboard memory **96** is also provided for storing data related to the usage of the system **80** and/or software for performing the various functions, etc.

Similarly, the injection component **84** in the embodiment illustrated in **Figure 3** includes a medicine reservoir **102** operatively connected to an injection mechanism **104** for delivering medication to the user. The injection component **84** can further include various electronics for enhancing the functions of the component. To this end, the illustrated injection component **84** includes a microprocessor **106** operatively connected to a user interface **108,** a communication interface **110,** and one or more sensors **112.** It will be appreciated, however, that the injection component **84** need not include any of the electrical components shown in **Figure 2****.** For example, the injection device can utilize a simple RFID transponder that is designed to be read by a corresponding RFID reader located in the wearable component **82,** such as in the system of **Figure 2****.**

The wearable component **82** can be configured to communicate with the injection component **84** to share information related to the operation of the system **80.** For example, the injection component **84** can communicate information such as the device ID, the patient ID, the medication, the dose volume, the recommended frequency for administering the medication, etc. Such information can be communicated to the wearable component **82** upon the first use of the injection component **84** with the wearable component **82.** A unique injection component identifier may also be provided to the wearable component **82** in order to allow the wearable component **82** to differentiate between different injection components **84.**

The wearable component **82** can be configured to sense or otherwise determine when an injection procedure has been performed and record data related to the injection procedure in the memory. For example, upon insertion of an injection component **84** into the wearable component **82,** the wearable component **82** may record the time and date of the sensed injection procedure. Combined with information received from the injection component, such as medicine type, dosage amounts, etc., a timeline can be constructed that illustrates a user's self administration of medication. The data used to construct such timeline may be transmitted to a third party via a variety of means. The wearable component may also provide scheduled prescription injection reminders to the user, for example through sight (e.g. blinking lights) or sound (e.g. audible alarm).

For example, and returning to **Figure 1****,** the wearable components described herein can communicate with a smartphone **16** via Bluetooth or other suitable connection to relay data from the wearable component to the smartphone. The smartphone **16** can then forward such information across the Internet or directly to a healthcare provider for review by a healthcare professional. By utilizing existing communication devices such as smart phones, personal computers and the like, the present disclosure facilitates real-time communication of information relating to patient self-administration of medicine to be monitored by health professionals thus improving patient medical history records and possibly patient compliance with treatment regimens.

Returning to **Figure 3****,** the wearable component **82,** as noted above, also includes a user interface **90.** The user interface **90** can include at least one of a user input device such as a button or buttons, a display and/or an audible alarm. The user interface **90** can be used for configuring the wearable component **82.** For example, a certain patient may need to administer a medication four times a day and the wearable component can be configured to remind a user approximately six hours after the last dose of medication was administered, to administer the next dose of medication. In the event that more than six hours lapses since the last administration of medication, the wearable component, having not sensed an injection procedure, may sound an alarm or otherwise indicate to the user the need to administer the next dose of medication. After an extended time lapses past the next scheduled dose, and if no injection procedure has been detected by the wearable component, the wearable component may send a signal to the smartphone which may be forwarded to a healthcare professional as noted informing the healthcare professional that the patient has missed their dose of medicine. The healthcare professional can then call or otherwise contact the user to determine the cause for the delay in administration of the scheduled dose.

As noted above, the injection component may simply contain an RFID tag. Such RFID tag may include a unique identifier and/or other information that allows the wearable component to determine certain information about the injection component. For example, the injection component could include an RFID tag having information regarding the type of medication and the dosage size of the medication. In such case, the injection component may be a single use disposable injection component where a user uses the component a single time injecting the entire payload of medication and then discards the injection component. In one embodiment, the wearable component can be configured to scan the RFID tag when an injection procedure is detected. To this end, a switch or other detection device may be provided within the opening of the housing of the wearable component. Such switch may be activated upon insertion of an injection component whereby an associated RFID tag reader in the wearable component would scan the RFID tag in the injection component and receive the information contained thereon.

The third party smart device **120** also includes a microprocessor **126** connected to a power supply **122,** which may be an onboard battery, AC power supply or the like. The smart device **120** also includes a user interface **128,** one or more sensors **124,** and a communication interface **130** for communicating with the wearable component **82.** Onboard memory **132** is also provided for storing data related to the usage of the system **80** and/or software for performing the various functions, etc.

As will now be appreciated, the present disclosure sets forth a wearable patch that can, among other things, apply pain management and track injection history, be used for multiple injections, provide reminders for scheduled injections (e.g., programmable), provide a target area for injection, and communicate information relating to usage of the device (e.g., injection history etc.) to health care personnel.

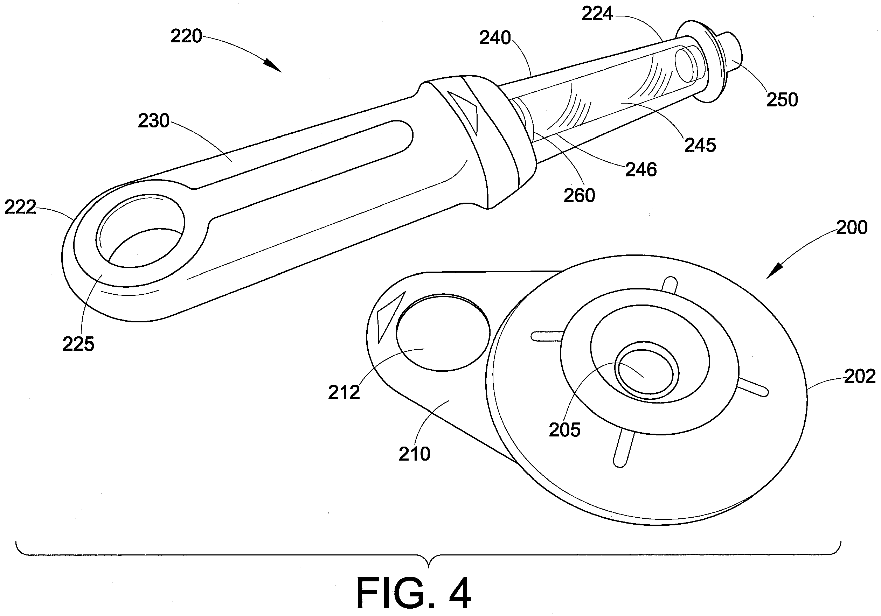

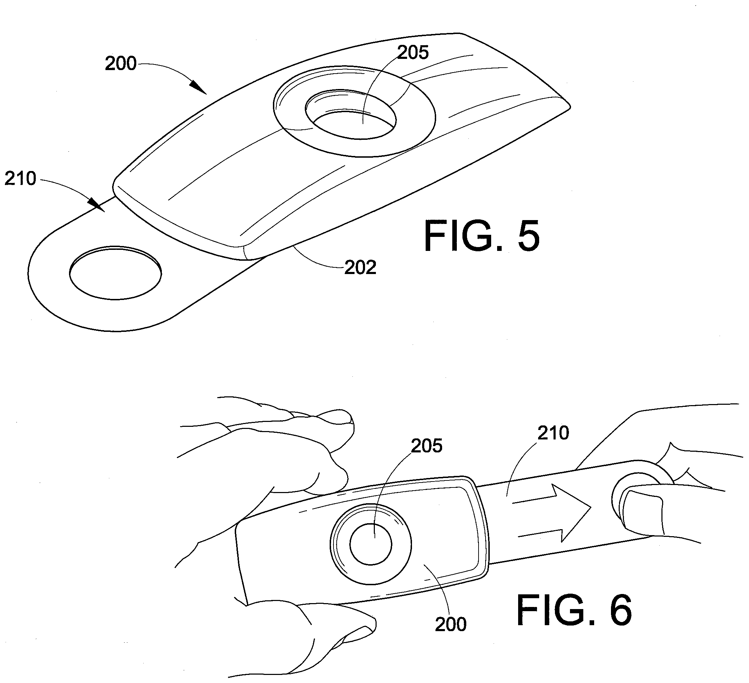

**Figure 4** is a perspective view of another exemplary medication administration system of the present disclosure. The injection device (i.e. syringe) and the patch device (i.e. wearable component) are visible here. Additional aspects of the system and the devices are visible in **Figures 5-8****.**

Turning first to the patch device **200,** the patch device again has a circular or disc-shaped perimeter **202.** An opening **205** is located in the patch device. The opening is contoured to be complementary to the needle end **224** of the injection device. A pull tab **210** is visible extending from one side of the perimeter of the patch device. The pull tab includes a large hole **212** through which the user's fingers can be inserted to grasp the pull tab and expose the adhesive on the bottom of the patch device for attachment to the skin. The pull tab itself can be made from a hypoallergenic plastic film or other known materials.

Referring now to **Figure 5**, a different embodiment of the patch device **200** is illustrated. Initially, this embodiment of the patch device has a rectangular perimeter **202.** The opening or injector interface **205** is again contoured to be complementary to the needle end of the injection device. The opening is in a central location. It is contemplated in some embodiments that the injector interface may contain an embedded magnet (not visible), which can be used to engage and secure the injection device during use. **Figure 6** is a top view showing the pull tab **210** being used to expose adhesive on the bottom of the patch device. The central opening **205** is also visible.

In embodiments, the patch device may have a diameter of from about 2 inches to about 3 inches. As may be recognized, this size permits, if desired, a number of such patch devices to be placed on the user's body. This can be useful in permitting the user to rotate the injection site being used, further reducing adverse reactions.

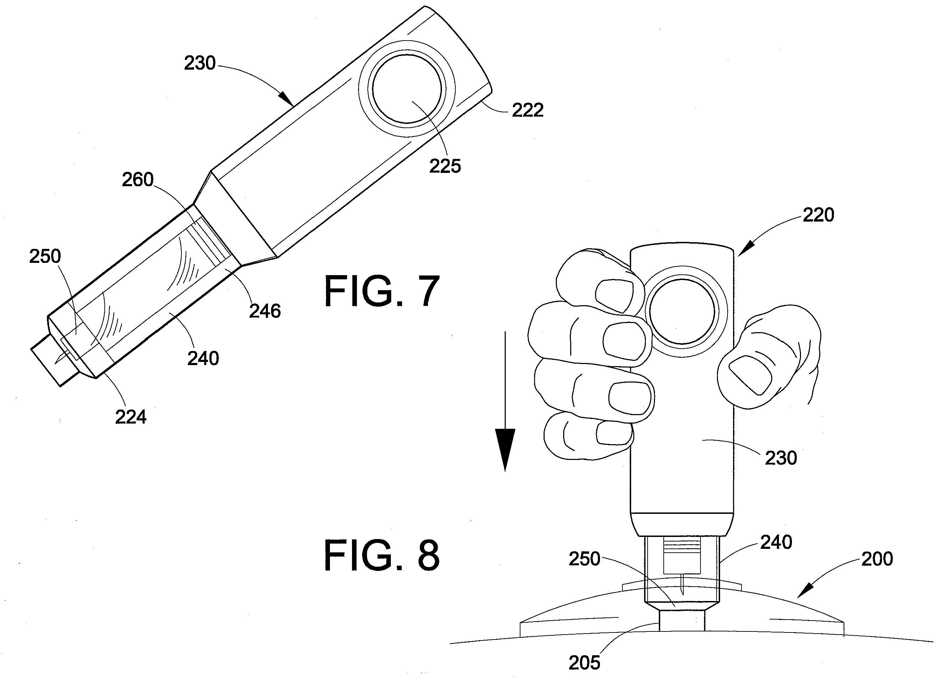

Referring now to **Figure 4** and **Figure 7**, the injection device **220** generally includes a handle **230,** a barrel **240,** and a base **250.** The injection device may be described as having a handle end **222** and a needle end **224.** A portion of the barrel is visible here, with the remainder of the barrel being located within the handle. The barrel is illustrated here as being transparent so that some inner components are visible.

The handle **230** is shaped to be useful to users with deficiencies and limitations in manual dexterity, coordination, and strength, for example users with symptoms of rheumatoid arthritis (RA), multiple sclerosis (MS), and other such conditions / diseases. For example, here the handle is sized to be easily grasped, and includes a ring-shaped grip 225. The base 250 of the injection device contains the needle (not visible) through which medication is dispensed. The base is also shaped to be complementary to the opening of the patch device. The barrel 240 includes the various components needed to dispense medication. For example, in Figure 7, a fluid chamber 245 is visible inside the barrel in which the medication is stored. The plunger 260 is located at the upper end 246 of the fluid chamber.

Figure 8 is a schematic illustration showing the injection device 220 and patch device 200 being used to dispense medication, with the patch device being shown in a cutaway. As seen here, the base 250 of the injection device interfaces with the opening 205 next to the user's skin. The handle 230 is then depressed downwards relative to the barrel 240 in order to activate the mechanism that depresses the plunger and delivers the medication. The needle (not visible) automatically inserts and retracts.

Materials and methods for making the various components of the systems disclosed herein are known in the art.

The processes and devices described herein may be used to deliver a high-viscosity fluid containing protein microparticles made using the processes described in U.S. Provisional Patent Application Serial No. No. 61/556,047, filed November 4, 2011. The devices described herein may also use the core annular flow processes and devices described in U.S. Provisional Patent Application Serial No. 61/556,491 , filed on November 7, 2011 , and in U.S. Provisional Patent Application Serial No. 61/673,864, filed on July 20, 2012. The devices described in U.S. Provisional Patent Application Serial No. No. 61/556,709, filed November 4, 2011, can also be used in the systems of the present disclosure.

The present disclosure has been described with reference to exemplary embodiments. Obviously, modifications and alterations will occur to others upon reading and understanding the preceding detailed description. It is intended that the present disclosure be construed as including all such modifications and alterations insofar as they come within the scope of the appended claims or the equivalents thereof.

## Claims

1. A medication administration system comprising:
a wearable component adapted to be worn on a surface of a user's skin, the wearable component including a housing having an opening, a cover coupled to the housing, and a flexible adhesive patch coupled to the housing for adhering the wearable component to the user's skin;
an injection component having an operative end configured to cooperate with the opening of the housing of the wearable component to administer a medication to the user, the wearable component including at least one monitoring device operative to monitor at least one parameter related to the use of at least one of the wearable component and the injection component, the cover of the wearable component being configured to cover at least a portion of the opening in the housing when the injection component is not received in the opening, the cover being configured to open upon insertion of the injection component into the opening.

2. The system of claim 1, wherein the injection component includes a pen-shaped structure.

3. The system of claim 1, wherein the patch contains pain management medication, and the patch is adapted to apply the pain management medication to an injection site of the user's skin before an injection.

4. The system of claim 3, wherein only a portion of the patch adjacent the opening contains the pain management medication.

5. The system of claim 1, wherein the cover comprises a plurality of flexible portions each secured about a circumference of the opening and each configured to deflect when the injection component is received in the opening to create an aperture through which the injection component delivers the medication.

6. The system of claim 1, wherein the operative end of the injection component includes a base containing a needle, the base being adapted to be received in the opening in the housing of the wearable component.

7. The system of claim 1, wherein the at least one monitoring device is operative to detect insertion of the injection component into the opening and to monitor treatment information provided with the injection component.

8. The system of claim 7, wherein the monitoring device includes a microprocessor in communication with at least one sensor for sensing a device identification and dose information associated with the injection component.

9. The system of claim 7, wherein the monitoring device includes a wireless communication interface for communicating with the injection component.

10. The system of claim 9, wherein the communication interface includes an RFID reader.

11. The system of claim 10, wherein the injection component includes an RFID transponder configured to be read by the RFID reader.

12. The system of claim 7, wherein the monitoring device includes a communication interface for communicating the treatment information to a third party device.

13. The system of claim 12, wherein the third party device includes at least one of a mobile phone, a computer, and an internet connected device.

14. The system of claim 12, further comprising the third party device.

15. The system of claim 1, wherein the wearable component includes a user interface having at least one of a user input device, a display, or a sound producing device.

16. The system of claim 1, wherein the injection component includes a medicine reservoir and an injection mechanism operative to inject the medication into a user's body.

17. The system of claim 16, wherein the at least one monitoring device comprises at least one sensor for detecting a parameter related to injection of the medication via the injection mechanism.

## Patentansprüche

1. Medikamentenverabreichungssystem, das Folgendes umfasst:
eine tragbare Komponente, die auf einer Hautoberfläche eines Benutzers getragen werden kann, wobei die tragbare Komponente Folgendes umfasst: ein Gehäuse mit einer Öffnung, einer Abdeckung, die mit dem Gehäuse gekoppelt ist, und ein flexibles Klebepflaster das mit dem Gehäuse gekoppelt ist, um die tragbare Komponente an der Haut des Benutzers anzubringen;
eine Injektionskomponente mit einem betriebsfähigen Ende, das dafür konfiguriert ist, mit der Öffnung des Gehäuses der tragbaren Komponente zusammenzuwirken, um dem Benutzer ein Medikament zu verabreichen, wobei die tragbare Komponente mindestens eine Überwachungsvorrichtung umfasst, die dazu dient, mindestens einen Parameter zu überwachen, der sich auf die Verwendung der tragbaren Komponente und/oder der Injektionskomponente bezieht, wobei die Abdeckung der tragbaren Komponente dafür konfiguriert ist, mindestens einen Abschnitt der Öffnung in dem Gehäuse zu bedecken, wenn die Injektionskomponente nicht in der Öffnung aufgenommen wird, wobei die Abdeckung dafür konfiguriert ist, sich beim Einführen der Injektionskomponente in die Öffnung zu öffnen.

2. System nach Anspruch 1, wobei die Injektionskomponente eine stiftförmige Struktur aufweist.

3. System nach Anspruch 1, wobei das Pflaster Schmerzmittel umfasst und das Pflaster dafür ausgelegt ist, das Schmerzmittel vor einer Injektion auf die Injektionsstelle der Haut des Benutzers aufzutragen.

4. System nach Anspruch 3, wobei nur ein Teil des Pflasters neben der Öffnung das Schmerzmittel umfasst.

5. System nach Anspruch 1, wobei die Abdeckung mehrere flexible Abschnitte aufweist, die jeweils um einen Umfang der Öffnung befestigt sind und die jeweils dafür konfiguriert sind, sich zu biegen, wenn die Injektionskomponente in der Öffnung aufgenommen wird, um eine Öffnung zu schaffen, durch die die Injektionskomponente das Medikament abgibt.

6. System nach Anspruch 1, wenn das betriebsfähige Ende der Injektionskomponente eine Basis umfasst, die eine Nadel umfasst, wobei die Basis dafür ausgelegt ist, in der Öffnung des Gehäuses der tragbaren Komponente aufgenommen zu werden.

7. System nach Anspruch 1, wobei die mindestens eine Überwachungsvorrichtung das Einführen der Injektionskomponente in die Öffnung erfassen und die Behandlungsinformationen, die mit der Injektionskomponente bereitgestellt werden, überwachen kann.

8. System nach Anspruch 7, wobei die Überwachungsvorrichtung einen Mikroprozessor in Kommunikation mit mindestens einem Sensor zum Erfassen von einer Vorrichtungsidentifikation und von Dosisinformationen umfasst, die der Injektionskomponente zugeordnet sind.

9. System nach Anspruch 7, wobei die Überwachungsvorrichtung eine drahtlose Kommunikationsschnittstelle zum Kommunizieren mit der Injektionskomponente umfasst.

10. System nach Anspruch 9, wobei die Kommunikationsschnittstelle einen RFID-Leser umfasst.

11. System nach Anspruch 10, wobei die Injektionskomponente einen RFID-Transponder umfasst, der dafür konfiguriert ist, von der RFID-Lesevorrichtung gelesen zu werden.

12. System nach Anspruch 7, wobei die Überwachungsvorrichtung eine Kommunikationsschnittstelle zum Übermitteln der Behandlungsinformationen an eine Vorrichtung eines Drittanbieters umfasst.

13. System nach Anspruch 12, wobei die Vorrichtung des Drittanbieters ein Mobiltelefon, einen Computer und/oder eine mit dem Internet verbundene Vorrichtung umfasst.

14. System nach Anspruch 12, das ferner die Vorrichtung des Drittanbieters umfasst.

15. System nach Anspruch 1, wobei die tragbare Komponente eine Benutzerschnittstelle mit einer Benutzereingabevorrichtung, einer Anzeige und/oder einer Tonerzeugungsvorrichtung umfasst.

16. System nach Anspruch 1, wobei die Injektionskomponente ein Medikamentenreservoir und einen Injektionsmechanismus umfasst, der dazu dient, das Medikament in den Körper eines Benutzers zu injizieren.

17. System nach Anspruch 16, wobei die mindestens eine Überwachungsvorrichtung mindestens einen Sensor zum Erfassen eines Parameters umfasst, der sich auf die Injektion des Medikaments über den Injektionsmechanismus bezieht.

## Revendications

1. Système d'administration de médicament, comprenant :
un composant portable adapté pour être porté sur une surface de la peau d'un utilisateur, le composant portable incluant un boîtier ayant une ouverture, un couvercle accouplé au boîtier, et un timbre adhésif flexible accouplé au boîtier pour faire adhérer le composant portable à la peau de l'utilisateur ;
un composant d'injection ayant une extrémité opérationnelle configurée pour coopérer avec l'ouverture du boîtier du composant portable pour administrer un médicament à l'utilisateur, le composant portable incluant au moins un dispositif de surveillance opérationnel pour surveiller au moins un paramètre connexe à l'utilisation d'au moins un du composant portable et du composant d'injection, le couvercle du composant portable étant configuré pour couvrir au moins une portion de l'ouverture dans le boîtier lorsque le composant d'injection n'est pas reçu dans l'ouverture, le couvercle étant configuré pour s'ouvrir lors de l'insertion du composant d'injection dans l'ouverture.

2. Système selon la revendication 1, dans lequel le composant d'injection inclut une structure en forme de stylo.

3. Système selon la revendication 1, dans lequel le timbre contient un médicament antidouleur, et le timbre est adapté pour appliquer le médicament antidouleur sur un site d'injection de la peau de l'utilisateur avant une injection.

4. Système selon la revendication 3, dans lequel seulement une portion du timbre adjacente à l'ouverture contient le médicament antidouleur.

5. Système selon la revendication 1, dans lequel le couvercle comprend une pluralité de portions flexibles, chacune fixée sur une circonférence de l'ouverture et chacune configurée pour se défléchir lorsque le composant d'injection est reçu dans l'ouverture pour créer un trou à travers lequel le composant d'injection administre le médicament.

6. Système selon la revendication 1, dans lequel l'extrémité opérationnelle du composant d'injection inclut une base contenant une aiguille, la base étant adaptée pour être reçue dans l'ouverture dans le boîtier du composant portable.

7. Système selon la revendication 1, dans lequel l'au moins un dispositif de surveillance est opérationnel pour détecter l'insertion du composant d'injection dans l'ouverture et pour surveiller des informations de traitement fournies avec le composant d'injection.

8. Système selon la revendication 7, dans lequel le dispositif de surveillance inclut un microprocesseur en communication avec au moins un capteur pour détecter une identification de dispositif et des informations de dose associées au composant d'injection.

9. Système selon la revendication 7, dans lequel le dispositif de surveillance inclut une interface de communication sans fil pour communiquer avec le composant d'injection.

10. Système selon la revendication 9, dans lequel l'interface de communication inclut un lecteur RFID.

11. Système selon la revendication 10, dans lequel le composant d'injection inclut un transpondeur RFID configuré pour être lu par le lecteur RFID.

12. Système selon la revendication 7, dans lequel le dispositif de surveillance inclut une interface de communication pour communiquer les informations de traitement à un dispositif tiers.

13. Système selon la revendication 12, dans lequel le dispositif tiers inclut au moins un d'un téléphone mobile, d'un ordinateur, et d'un dispositif connecté à Internet.

14. Système selon la revendication 12, comprenant en outre le dispositif tiers.

15. Système selon la revendication 1, dans lequel le composant portable inclut une interface utilisateur ayant au moins un d'un dispositif d'entrée utilisateur, d'une unité d'affichage, ou d'un dispositif sonore.

16. Système selon la revendication 1, dans lequel le composant d'injection inclut un réservoir de médicament et un mécanisme d'injection opérationnel pour injecter le médicament dans le corps d'un utilisateur.

17. Système selon la revendication 16, dans lequel l'au moins un dispositif de surveillance comprend au moins un capteur pour détecter un paramètre connexe à l'injection du médicament par l'intermédiaire du mécanisme d'injection.
